# EUROPEAN PATENT SPECIFICATION

(11) **EP 1 422 159 B1**
(45) Date of publication and mention of the grant of the patent: **13.06.2007**
(21) Application number: 03009544.2
(22) Date of filing: 28.04.2003
(51) Int. Cl.: B65D 75/38, B65D 75/58, B65D 81/32

(54) **Sealed container**
Versiegelter Behälter
Récipient scellé

(30) Priority: 25.11.2002 US 303330
(43) Date of publication of application: 26.05.2004
(73) Proprietor: Tsaur, Garry, Rowland Heights, CA 91748 (US)
(72) Inventor: Tsaur, Garry, Rowland Heights, CA 91748 (US)
(74) Representative: Kühn, Armin

(56) References cited:
- EP-A- 0 622 304
- US-A- 3 918 435
- US-A- 4 863 422
- US-A- 4 903 708
- US-A- 5 490 736
- US-B1- 6 467 982

## Description

### BACKGROUND-FIELD OF INVENTION

The present invention relates to a sealed container. More specifically, the present invention relates to a sealed container that may be used as the body of a cotton swab.

### BACKGROUND-DESCRIPTION OF RELATED ART

Containers that enclose fluids to be stored and transported are often inconvenient to use and most requires an applicator or other tools to extract the fluid. Storage of fluids such as solvents and alcohol present a special consideration due to the evaporation of the fluids. Various designs exist for various applications. Containers may be as large as a tank for storing liquids such as water. Containers may also be as small as bottles for perfumes or smaller. Containers may also be made of various suitable materials for any particular environment and liquid.

US-A-4 903 708 discloses a swab transport apparatus for containing and transporting a swab having an elongated stem and a bibulous swab material carried proximal one end of the stem, said apparatus comprising an elongated plastic body having a longitudinally extending axis, resiliently deformable side walls and first and second ends, said body being open at said first end and including a first elongated chamber located proximate to and communicating with said first open end for receiving at least a portion of the swab; and a second chamber having resiliently, deformable side walls, said second chamber being axially offset and longitudinally displaced from the said first chamber.

EP 0 622 304 A1 discloses a blow molded plastic container containing a handgrip comprising: a neck portion defining an opening, a bottom portion and a body portion interconnecting said neck and bottom portions, wherein the neck, body and bottom portions define a hollow space closed at the bottom portion and open at the neck portions; said neck, body and bottom portions having an inside wall face and an outside wall face; at least one supporting member in the hollow space extending completely across the hollow space and integral with the inside wall face of the body portion at two spaced locations thereof to support said inside wall face.

The present invention is a small elongated sealed container that can be used as the body of a cotton swab and also for storing fluids wherein the fluid in the sealed container may be released into the cottons attached to the ends of the cotton swab when desired. The sealed container of the present invention is particularly suited for the storage of solvents, alcohol, and other fluids that evaporates easily.

### SUMMARY OF THE INVENTION

To this end, the invention provides a sealed container comprising the features of claim 1. Further embodiments of the invention are described in the dependent claims.

The present invention is a sealed container that stores fluid in a sealed environment and is easily transportable. The contents of the sealed container can be easily extracted completely without any tools or contact with the user. The sealed container can be used as the body of a cotton swab and also for storing fluids wherein the fluid in the sealed container may be released into the cottons attached to the ends of the cotton swab when desired.

### BRIEF DESCRIPTION OF THE DRAWINGS

Figure 1 shows the preferred embodiment of the sealed container with two opening means in the form of scores on the sealed container with an elongated tube that is open at both ends and is smaller in diameter and shorter in length than the sealed container inserted into the sealed container separating the sealed container into two cylindrical compartments.
Figure 2 shows another embodiment of the sealed container with two opening means in the form of scores on the sealed container with a divider separating the sealed container into two compartments.

### DETAILED DESCRIPTION OF THE PREFERRED EMBODIMENT

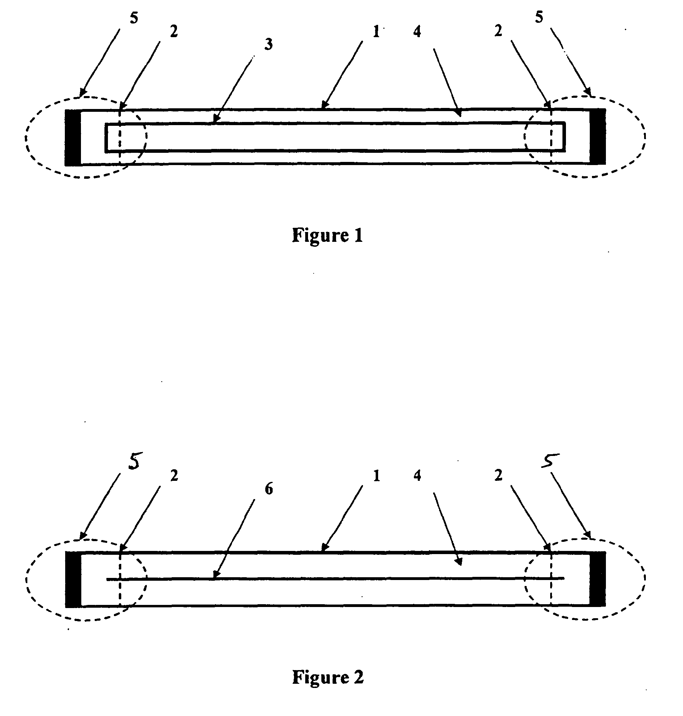

Figure 1 shows the preferred embodiment of the sealed container. The sealed container has an elongated housing **1** with two ends that are sealed. An opening means **2** in the form of a score on the sealed container is located near each of the two ends. An elongated tube **3** that is open at both ends and is smaller in diameter and shorter in length than the elongated housing **1** is inserted into the elongated housing **1** separating the elongated housing **1** into two cylindrical compartments. A fluid **4** is sealed within the elongated housing **1** until released through the opening means **2**. In the preferred embodiment, a cotton ball **5** is attached to the ends of the sealed container enclosing the opening means **2** to absorb the fluid **4** when it is released from the sealed container.

Figure 2 shows another embodiment of the sealed container wherein the elongated housing **1** is divided into two compartments by a divider **6** that span across the width of the elongated housing **1** and is shorter than the length of the elongated housing **1** and which allows the movement of fluids **4** between the two compartments within the elongated housing **1** at both ends of the elongated housing **1**.

The sealed container of the present invention allows a fluid **4** that evaporates easily, such as solvents and alcohol, to be stored in a sealed environment to prevent evaporation. The fluid **4** may be released easily through either one of the opening means **2** by simply breaking open the end at the scoring to allow the fluid **4** to flow into the cotton ball **5**. Without the two compartment design of the present invention, the fluid **4** within the sealed container will not be released from the sealed container due to the small diameter of the sealed container and the resulting capillary action and atmospheric pressure, which will prevent the exit of the fluids **4** from the sealed container.

Although the description above contains many specificities, these should not be construed as limiting the scope of the invention but as merely providing illustrations of some of the presently preferred embodiments of this invention. Thus the scope of the invention should be determined by the appended claims, rather than by the examples given.

## Claims

1. A sealed container comprising:
an elongated housing (1) with two longitudinal ends which are sealed and with one or more opening means (2);
a divider (3; 6) longitudinally separating the elongated housing (1) into a first continuous compartment and a second continuous compartment, the divider (3; 6) being shorter than the elongate housing (1) in manner to allow free flow of fluid (4) between the first and the second continuous compartment at both longitudinal ends of the elongated housing (1);
wherein the sealed container is used to store fluid (4) until the fluid (4) is released through the respective opening means (2).

2. The sealed container as in claim 1, wherein the divider (3; 6) is an elongated tube (3) thereby providing both the first and the second compartments as cylindrical compartments.

3. The sealed container as in claim 1 or 2, wherein the opening means (2) is a scoring that can be broken open to release the fluid (4) in the sealed container.

4. The sealed container as in one of claims 1 to 3, wherein the fluid (4) is alcohol.

5. A sealed container as in one of claims 1 to 4, wherein the opening means (2) is covered with an absorbent material such as a ball of cotton (5) or a sponge.

## Patentansprüche

1. Versiegelter Behälter, umfassend:
ein längliches Gehäuse (1) mit zwei versiegelten Längsenden und mit einem oder mehreren Öffnungsmitteln (2),
ein Unterteilungsteil (3, 6), das das längliche Gehäuse (1) der Länge nach in einen ersten durchgehenden Raum und einen zweiten durchgehenden Raum aufteilt, wobei das Unterteilungsteil (3, 6) derart kürzer als das längliche Gehäuse (1) ist, um einen freien Fluss von Flüssigkeit (4) zwischen dem ersten und dem zweiten durchgehenden Raum an beiden Längsenden des länglichen Gehäuses (1) zu ermöglichen,
wobei der versiegelte Behälter verwendet wird, um Flüssigkeit (4) zu bevorraten, bis die Flüssigkeit (4) durch das entsprechende Öffnungsmittel (2) freigegeben wird.

2. Versiegelter Behälter nach Anspruch 1, wobei das Unterteilungsteil (3, 6) ein längliches Röhrchen (3) ist, wodurch sowohl der erste als auch der zweite Raum als zylinderförmige Räume ausgebildet sind.

3. Versiegelter Behälter nach Anspruch 1 oder 2, wobei das Öffnungsmittel (2) eine Schwächungslinie ist, die aufgebrochen werden kann, um die Flüssigkeit (4) in dem versiegelten Behälter freizugeben.

4. Versiegelter Behälter nach einem der Ansprüche 1 bis 3, wobei die Flüssigkeit (4) Alkohol ist.

5. Versiegelter Behälter nach einem der Ansprüche 1 bis 4, wobei das Öffnungsmittel (2) mit einem absorbierenden Material, wie einem Wattebausch (5) oder einem Schwamm, umgeben ist.

## Revendications

1. Récipient scellé comprenant :
un logement allongé (1) muni de deux extrémités longitudinales qui sont scellées et équipées d'un ou plusieurs dispositifs d'ouverture (2) ;
un séparateur (3 ; 6) séparant de manière longitudinale le logement allongé (1) en un premier compartiment continu et un second compartiment continu, le séparateur (3 ; 6) étant plus court que le logement allongé (1) de manière à permettre au fluide (4) de circuler librement entre le premier et le second compartiment continu au niveau des deux extrémités longitudinales du logement allongé (1) ;
dans lequel le récipient scellé est utilisé pour stocker du fluide (4) jusqu'à ce que le fluide (4) soit libéré à travers les dispositifs d'ouverture respectifs (2).

2. Récipient scellé selon la revendication 1 dans lequel le séparateur (3 ; 6) est un tube allongé (3) de sorte que les premier et second compartiments sont des compartiments cylindriques.

3. Récipient scellé selon la revendication 1 ou 2, dans lequel le dispositif d'ouverture (2) est une entaille qui peut être ouverte par force pour libérer le fluide (4) dans le récipient scellé.

4. Récipient scellé selon l'une des revendications 1 à 3, dans lequel le fluide (4) est de l'alcool.

5. Récipient scellé selon l'une des revendications 1 à 4, dans lequel le dispositif d'ouverture (2) est recouvert par un matériau absorbant comme une pelote de coton (5) ou une éponge.
